# EUROPEAN PATENT SPECIFICATION

(11) **EP 2 229 919 B1**
(45) Date of publication and mention of the grant of the patent: **21.01.2015**
(21) Application number: 10006773.5
(22) Date of filing: 04.03.2005
(51) Int. Cl.: A61F 2/82, A61F 2/91

(54) **Anti-restenotic drug for use in method with bioresorbable stent with reservoirs**
Antirestenotisches Medikament zur Verwendung im Verfahren mit bioresorbierbarem Stent mit Reservoirs
Médicament anti-resténose pour l'utilisation dans un procédé avec une endoprothèse biorésorbable à réservoirs

(30) Priority: 08.04.2004 US 822063
(43) Date of publication of application: 22.09.2010
(62) Divisional of application: 05729863.0
(73) Proprietor: Innovational Holdings, LLC, New Brunswick, NJ 08933 (US)
(72) Inventor: Shanley, John F., Menlo Park, CA 94025 (US); Litvack, Frank, Menlo Park, CA 94025 (US); Parker, Theodore L., Menlo Park, CA 94025 (US); Diaz, Stephen H., Menlo Park, CA 94025 (US)
(74) Representative: Diehl & Partner GbR

(56) References cited:
- US-A1- 2002 007 209
- US-A1- 2002 082 680
- US-B1- 6 306 166

## Description

### Background

Most coronary artery-related deaths are caused by atherosclerotic lesions which limit or obstruct coronary blood flow to heart tissue. To address coronary artery disease, doctors often resort to percutaneous transluminal coronary angioplasty (PTCA) or coronary artery bypass graft (CABG). PTCA is a procedure in which a small balloon catheter is passed down a narrowed coronary artery and then expanded to re-open the artery. The major advantage of angioplasty is that patients in which the procedure is successful need not undergo the more invasive surgical procedure of coronary artery bypass graft. A major difficulty with PTCA is the problem of post-angioplasty closure of the vessel, both immediately after PTCA (acute reocclusion) and in the long term (restenosis).

Coronary stents are typically used in combination with PTCA to reduce reocclusion of the artery. Stents are introduced percutaneously, and transported transluminally until positioned at a desired location. These devices are then expanded either mechanically, such as by the expansion of a mandrel or balloon positioned inside the device, or expand themselves by releasing stored energy upon actuation within the body. Once expanded within the lumen, these devices, called stents, become encapsulated within the body tissue and remain a permanent implant.

Restenosis is a major complication that can arise following vascular interventions such as angioplasty and the implantation of stents. Simply defined, restenosis is a wound healing process that reduces the vessel lumen diameter by extracellular matrix deposition, neointimal hyperplasia, and vascular smooth muscle cell proliferation, and which may ultimately result in renarrowing or even reocclusion of the lumen. Despite the introduction of improved surgical techniques, devices, and pharmaceutical agents, the overall restenosis rate is still reported in the range of 25% to 50% within six to twelve months after an angioplasty procedure. To treat this condition, additional revascularization procedures are frequently required, thereby increasing trauma and risk to the patient.

While the exact mechanisms of restenosis are still being determined, certain agents have been demonstrated to reduce restenosis in humans. One example of an agent which has been demonstrated to reduce restenosis when delivered from a stent is paclitaxel, a well-known compound that is commonly used in the treatment of cancerous tumors. However, the stents which are currently available and under development for delivery of anti-restenotic agents use surface coatings with suboptimal agent release profiles and side effects. In one example, over 90% of the total agent loaded onto the stent is permanently retained in the stent and is never delivered to the tissue.

There are two types of stents that are presently utilized: permanent stents and bioresorbable stents. A permanent stent is designed to be maintained in a body lumen for an indeterminate amount of time. Permanent stents are typically designed to provide long-term support for damaged or traumatized wall tissues of the lumen. There are numerous conventional applications for permanent stents including cardiovascular, peripheral, urological, gastrointestinal, and gynecological applications.

Bioresorbable stents may advantageously be eliminated from body lumens after a predetermined, clinically appropriate period of time, for example, after the traumatized tissues of the lumen have healed and a stent is no longer needed to maintain the patency of the lumen.

It is known that the metal stents may become encrusted, encapsulated, endothelialized or ingrown with body tissue. Metal stents could possibly cause irritation to the surrounding tissues in a lumen due to the fact that metals are typically much harder and stiffer than the surrounding tissues in a lumen, which may result in an anatomical or physiological mismatch, thereby damaging tissue or eliciting unwanted biologic responses.

It is known to use bioabsorbable and bioresorbable materials for manufacturing stents. The conventional bioabsorbable or bioresorbable materials from which such stents are made are selected to resorb or degrade over time, thereby eliminating the need for subsequent surgical procedures to remove the stent from the body lumen if problems arise. However, formation of a bioabsorbable stent with a drug within the stent is difficult because the thermoforming processes necessary for formation of the bioabsorbable stent are often not tolerated by the drug. Further, as discussed above, surface coatings on bioabsorbable stents, like the coatings on permanent metal stents have difficulty in controlling the release of the drug due to the limitations of a surface coating.

It is further known from US 2002/0007209 A1 to use an anti-restenotic drug in a method of reducing restenosis, providing a drug delivery bioresorbable stent with a dosage of the anti-restenotic drug in a bioresorbable polymer matrix arranged within a plurality of opening in the stent.

### Summary of the Invention

The present invention relates to an anti-restenotic drug for use in a method of reducing restenosis with a bioresorbable drug delivery stent comprising a substantially cylindrical expandable stent formed of a bioresorbable material and a plurality of reservoirs formed in the stent containing a beneficial agent matrix comprising a bioresorbable polymer and a drug, arranged within a plurality of openings in the stent without coating an exterior surface of the stent with the anti-restenotic drug, wherein the bioresorbable material of the stent degrades more slowly than the bioresorbable polymer matrix and the bioresorbable polymer matrix is arranged in the plurality of openings such that it does not change the bioresorption of the stent.

a bioresorbable drug delivery stent includes a substantially cylindrical expandable stent formed of a plurality of struts of a bioresorbable material, a plurality of openings formed in the stent struts, and a beneficial agent matrix loaded within the plurality of openings, the beneficial agent matrix comprising a bioresorbable matrix material drug.

a bioresorbable drug delivery stent includes a substantially cylindrical expandable stent body formed of a bioresorbable material and a plurality of openings formed in the stent body containing a beneficial agent matrix comprising a bioresorbable polymer and a drug, wherein the bioresorbable material of the stent body is a different material than the bioresorbable polymer of the beneficial agent matrix.

In accordance with the present disclosure, a method of reducing restenosis with a bioresorbable drug delivery stent, includes the steps of providing a drug delivery bioresorbable stent having a dosage of anti-restenotic drug arranged within a plurality of openings in the stent without coating an exterior surface of the stent with the anti-restenotic drug, implanting the stent within an artery of a patient, and delivering the anti-restenotic drug from the stent to the artery at a minimum release rate of 1 percent of the total dosage of the drug on the stent per day throughout an entire administration period from the time of implantation of the stent until the time that substantially all the drug is released from the stent.

Further In accordance with the present disclosure, a bioresorbable drug delivery stent includes a substantially cylindrical expandable stent formed of a bioresorbable material, a plurality of openings formed in the stent, and a beneficial agent matrix loaded within the plurality of openings, the beneficial agent matrix comprising a drug. The beneficial agent matrix is arranged such that the beneficial agent matrix does not block access of fluid from an environment surrounding the stent to the bioresorbable stent material.

### Brief Description of the Drawings

The invention will now be described in greater detail with reference to the accompanying drawings, in which like elements bear like reference numerals, and wherein:
FIG. 1 is a perspective view of one example of a stent according to the present disclosure.
FIG. 2 is a side view of a portion of the stent of FIG. 1.
FIG. 3 is a side view of a portion of another example of a stent woven from filaments.
FIG. 4 is a side view of a portion of another example of a stent with a lattice configuration.
FIG. 5 is a side cross sectional view of an example of an opening in a stent showing a matrix with a therapeutic agent and a barrier layer.
FIG. 6 is a side cross sectional view of another example of an opening in a stent showing a matrix with two therapeutic agents.

### Detailed Description

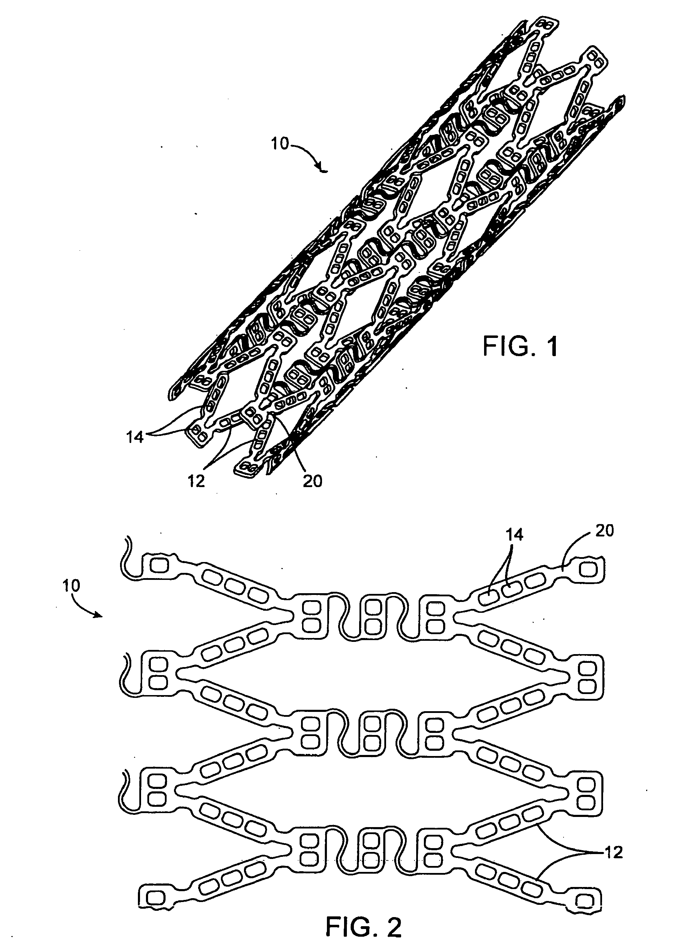

A biodegradable or bioresorbable drug delivery stent as illustrated in FIGS. 1- 4 of the present disclosure includes a substantially cylindrical expandable stent formed of a bioresorbable material and a plurality of reservoirs formed in the stent containing a beneficial agent matrix. The bioresorbable stent material can be a bioresorbable metal alloy, a bioresorbable polymer, a bioresorbable composite or the like which has sufficient structural integrity to support a lumen, such as a blood vessel lumen for a predetermined period of time. The reservoirs containing the beneficial agent matrix allow delivery of the beneficial agent, such as an antirestenotic drug, for an administration period which is generally equal to or less than a time that the bioresorbable stent is retained in the lumen. The beneficial agent matrix may include one or more bioresorbable polymers or other matrix materials in combination with one or more therapeutic agents or drugs.

The following terms, as used herein, shall have the following meanings:
The terms "drug" and "therapeutic agent" are used interchangeably to refer to any therapeutically active substance that is delivered to a living being to produce a desired, usually beneficial, effect.
The term "beneficial agent" as used herein is intended to have its broadest possible interpretation and is used to include any therapeutic agent or drug, as well as inactive agents such as barrier layers, carrier layers, therapeutic layers, or protective layers.
The term "matrix" or "biocompatible matrix" are used interchangeably to refer to a medium or material that, upon implantation in a subject, does not elicit a detrimental response sufficient to result in the rejection of the matrix. The matrix may contain or surround a therapeutic agent, and/or modulate the release of the therapeutic agent into the body. A matrix is also a medium that may simply provide support, structural integrity or structural barriers. The matrix may be polymeric, non-polymeric, hydrophobic, hydrophilic, lipophilic, amphiphilic, crystalline and the like.
The term "bioresorbable" refers to a material, as defined herein, that can be broken down by either chemical or physical process, upon interaction with a physiological environment. The bioresorbable material can erode or dissolve. A bioresorbable material serves a temporary function in the body, such as supporting a lumen or drug delivery, and is then degraded or broken into components that are metabolizable or excretable, over a period of time from minutes to years, preferably less than one year, while maintaining any requisite structural integrity in that same time period.
The term "openings" includes both through openings and recesses.
The term "pharmaceutically acceptable" refers to the characteristic of being non-toxic to a host or patient and suitable for maintaining the stability of a therapeutic agent and allowing the delivery of the therapeutic agent to target cells or tissue.
The term "polymer" refers to molecules formed from the chemical union of two or more repeating units, called monomers. Accordingly, included within the term "polymer" may be, for example, dimers, trimers and oligomers. The polymer may be synthetic, naturally-occurring or semisynthetic. In preferred form, the term "polymer" refers to molecules which typically have a M_{W} greater than about 3000 and preferably greater than about 10,000 and a M_{W} that is less than about 10 million, preferably less than about a million and more preferably less than about 200,000. Examples of polymers include but are not limited to, poly-α-hydroxy acid esters such as, polylactic acid (PLLA or DLPLA), polyglycolic acid, polylactic-co-glycolic acid (PLGA), polylactic acid-co-caprolactone; poly (block-ethylene oxide-block-lactide-co-glycolide) polymers (PEO-block-PLGA and PEO-block-PLGA-block-PEO); polyethylene glycol and polyethylene oxide, poly (block-ethylene oxide-block-propylene oxide-block-ethylene oxide); polyvinyl pyrrolidone; polyorthoesters; polysaccharides and polysaccharide derivatives such as polyhyaluronic acid, poly (glucose), polyalginic acid, chitin, chitosan, chitosan derivatives, cellulose, methyl cellulose, hydroxyethylcellulose, hydroxypropylcellulose, carboxymethylcellulose, cyclodextrins and substituted cyclodextrins, such as beta-cyclodextrin sulfobutyl ethers; polypeptides and proteins, such as polylysine, polyglutamic acid, albumin; polyanhydrides; polyhydroxy alkonoates such as polyhydroxy valerate, polyhydroxy butyrate, and the like.
The term "primarily" with respect to directional delivery, refers to an amount greater than about 50% of the total amount of therapeutic agent provided to a blood vessel.
The term "restenosis" refers to the renarrowing of an artery following an angioplasty procedure which may include stenosis following stent implantation.
The term "substantially linear release profile" refers to a release profile defined by a plot of the cumulative drug released versus the time during which the release takes place in which the linear least squares fit of such a release profile plot has a correlation coefficient value, r², of greater than 0.92 for data time points after the first day of delivery.

FIG. 1 illustrates one example of an implantable medical device in the form of a biodegradable or bioresorbable stent 10. FIG. 2 is an enlarged flattened view of a portion of the stent of FIG. 1 illustrating one example of a stent structure including struts 12 interconnected by ductile hinges 20. The struts 12 include openings 14 which can be non-deforming openings containing a therapeutic agent. One example of a stent structure having non-deforming openings is shown in U.S. Patent No. 6,562,065 .

The bioresorbable stent 10 can be formed of a bioresorbable metal alloy, a bioresorbable polymer. Bioresorbable metal alloys useful for stents include zinc-titanium alloys, and magnesium alloys, such as lithium-magnesium, sodium-magnesium, and magnesium alloys containing rare earth metals. Some examples of bioresorbable metal alloys are described in U.S. Patent No. 6,287,332. Bioresorbable metal alloy stents can be formed in the configuration illustrated in FIGS. 1 and 2 by laser cutting. When cutting stents from these alloys, an inert atmosphere may be desired to minimize oxidation of the alloy during cutting in which case, a helium gas stream, or other inert atmosphere can be applied during cutting. Magnesium alloys are used in the aeronautic industry and the processing systems used for the aeronautic industry can also be used for forming the stents. Bioresorbable metal alloys provide the necessary structural strength needed for the stent, however, it is difficult to incorporate a drug within the bioresorbable metal alloy and is difficult to release the drug if it could be incorporated.

More importantly, the use of coatings on the bioresorbable metal alloy surface containing a drug may interfere with the biodegradation of the stent. Therefore, the present invention of providing openings in the bioresorbable stent and filling the openings with a bioresorbable matrix containing drug provides a solution because there is no requirement for a coating on the stent.

When the bioresorbable stent 10 is formed of a bioresorbable polymer material, similar problems can occur when attempting to adding a drug to the stent by incorporating drug into the polymer or coating drug onto the stent. For example, bioresorbable polymers which have sufficient strength to be used as a stent may not be capable of incorporating a drug and releasing the drug in a desired manner. Further, drug coatings require that they adhere well without cracking or flaking during delivery and also release the drug in a desired manner. Additionally, polymer stents tend to have high recoil.

Another difficulty in incorporating drugs in polymer stents is that methods for forming bioresorbable polymer stents tend to be high temperature processes which are not suitable for many drugs. With polymer stents, as with bioresorbable metal alloys, a coating may also interfere with bioresorbtion of the stent.

The bioresorbable stent of the present application provides a solution to these problems by selecting a first bioresorbable polymer for the struts of the stent and providing openings in the stent containing a beneficial agent matrix. The polymer or other matrix material in the openings require none of the structural properties of the stent, and also require very little flexibility or adhesion which is required by a coating. Thus, the matrix material selection may be made based on the ability of the material to release the drug with a desired release profile. Directional delivery of one or more drugs can also be achieved with reservoirs which cannot be easily achieved with coatings, impregnation, or other methods.

Examples of bioresorbable polymers which can be used for the structural struts of the stent 10 include, without limitation, polylactic acid (PLA), polyglycolic acid (PGA), copolymers of PLA and PGA, poly-L-lactide (PLLA), poly-D,L-lactide (PDLA), poly-e-capralactone (PCL), and combinations thereof. U.S. Patent No. 4,889,119, describes some of the bioresorbable polymers which are useful in the present invention.

Examples ofbioresorbable polymers which can be used for the polymer/drug matrix within the reservoirs include, without limitation, polylactic acid (PLA); polyglycolic acid (PGA); copolymers of PLA and PGA; polylactic-co-glycolic acid (PLGA); poly-L-lactide (PLLA); poly-D,L-lactide (PDLA); poly-ε capralactone (PCL); polyethylene glycol and polyethylene oxide, poly (block-ethylene oxide-block-propylene oxide-block-ethylene oxide); polyvinyl pyrrolidone; polyorthoesters; polysaccharides and polysaccharide derivatives such as polyhyaluronic acid, poly (glucose), polyalginic acid, chitin, chitosan, chitosan derivatives, cellulose, methyl cellulose, hydroxyethylcellulose, hydroxypropylcellulose, carboxymethylcellulose, cyclodextrins and substituted cyclodextrins, such as beta-cyclodextrin sulfobutyl ethers; polypeptides and proteins, such as polylysine, polyglutamic acid, albumin; and combinations thereof. Preferably, the polymer in the reservoir degrades at a rate which results in degradation of the matrix substantially at the same time or before the degradation of the stent itself.

Bioresorbable polymer stents can be formed by known methods including molding, extrusion, other thermoforming processes, laser cutting, semiconductor fabrication methods including microdischarge machining or a combination of these processes. Laser cutting of a polymer tube to form a stent 10, such as the stent illustrated in FIGS. 1 and 2, can be performed with a UV laser, excimer laser or other known laser. The stent illustrated in FIGS. 1 and 2 is only one example of the type of stent structure which may be made. Many other stent configurations can also be used including woven stents, coil stents, serpentine patterns, diamond patterns, chevron or other patterns, or racheting or locking stents.

Molds for forming bioresorbable polymer stents can be formed by a number of know methods including photolithography, EMD, other semiconductor fabrication processes, degradable molds, lost wax casting, or the like. For example, in one process, a stent form can be created by photolithography, a silicon rubber mold can be formed from the stent form, and the rubber mold can be metalized to created the rigid stent mold useful for molding the polymer stents under high pressure. The stent 10 can be molded with the openings 14 formed during the molding step. Alternatively, the openings 14 can be formed in a later step, such as by laser cutting.

The mold used to form the stent may include a central pin or core and two or more surrounding removable mold members. The molded stents can be removed from the core by one of several methods including mechanically by lifting pins or wires, pneumatically by passage of air under the stents, or by swelling the plastic by application of a liquid, such as a solvent to a swellable material, such as a cross-linked polymer. Alternatively, the core can be formed of a collapsible configuration.

Although the openings 14 have been illustrated as through holes, other shaped openings including recesses, channels, wells, and grooves can be easily formed by a molding process.

Although similar bioresorbable polymers can be used for the stent structure and the polymer/drug matrix in the openings, these polymers are formed in different ways. The stent polymer is formed by a high temperature forming process, for example, temperatures of above 100 degrees C and preferably above 120 degrees C can be required for forming the stent. However, since these high temperatures cause degradation of most drugs, the polymer of the polymer/drug matrix is formed by a different process, such as with the use of a solvent at a lower temperature which is generally below 100 degrees C, and preferably below about 75 degrees C. The present invention separates the step of forming the structural portion of the stent from the step of forming the drug delivery portion of the stent without requiring a coating.

The bioresorbable material of the matrix and any other materials within the reservoirs can be delivered into the openings in a liquidified state which can be achieved by either a solvent or an elevated temperature. When a solvent is used to deliver the matrix solution into the openings, the solvent selected should be a solvent which does not substantially degrade the bioresorbable material of the stent. For example, a stent formed of PLLA can be formed with openings which can be filled with a solution comprising PLGA, DMSO, and drug. The DMSO will not appreciably degrade the PLLA of the stent and will be evaporated to form the polymer/drug matrix within the openings. In another example, the polymer of the stent can be cross-linked, coated, or otherwise treated to prevent the solvent from degrading the polymer.

In a further example, a stent formed of PLGA can include openings which are filled with a hydrophilic polymer (PEO, PVP, dextrin) and a hydrophilic drug (insulin) dissolved in water.

The bioresorbable polymer and bioresorbable metal alloy stents can be either balloon expandable or self expanding. For example, self expanding polymer stents may be formed in an expanded configuration and compressed for delivery within a delivery system which constrains the stent. When the delivery system constrains are removed, the stent returns to the expanded size. In another example, a self expanding polymer stent can be retained on a balloon catheter by a breakable or erodible constraining mechanism, such as a thread. Upon delivery of the balloon catheter to a desired implantation position within a lumen, the balloon is expanded, thus breaking the thread and allowing the stent to expand to support the lumen.

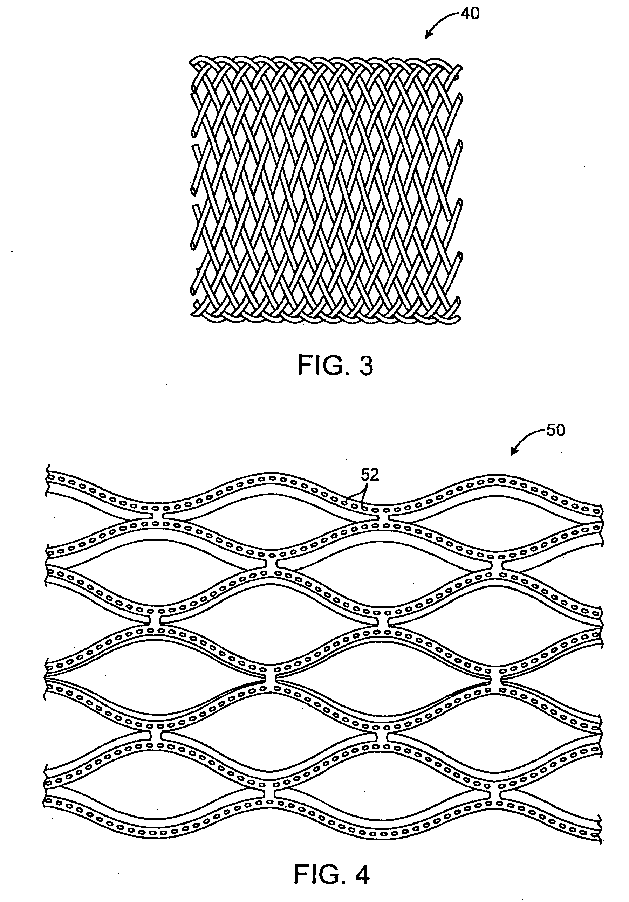

FIG. 3 illustrates an alternative example of a bioresorbable stent 40 which is woven from a bioresorbable wire. The bioresorbable wire may be any of the bioresorbable metal alloys, bioresorbable polymer materials, or other bioresorbable materials described above. In the mesh stent, reservoirs are formed in the wires of the mesh either before or after weaving the wires into the mesh. The reservoirs can also be filled with the polymer/drug matrix either before or after weaving.

In a second example, the bioresorbable wire mesh stent 40 of FIG. 3 can be woven and then compressed under application of heat to form the mesh into a single layer of lattice with gaps or diamond shaped openings between the lattice members. These gaps or openings are then filled with the bioresorbable drug delivery matrix to form the drug delivery stent.

FIG. 4 illustrates another example of a bioresorbable stent 50 which can be extruded, molded, or laser cut in a lattice structure. The openings 52 can be formed in the lattice structure of the stent 50 either during the process of forming the stent or subsequently. The openings 52 are then filled with the polymer/drug matrix.

### The Beneficial Agent Matrix Formation

The bioresorbable stents of the present invention are configured to release at least one therapeutic agent from the matrix contained in reservoirs in the implantable stent body. The matrix is formed such that the distribution of the agent in the polymer matrix as well as barrier layers, protective layers, separating layers, and cap layers which form a part of the matrix together control the rate of elution of the agent from the reservoirs.

In one embodiment, the matrix is a polymeric material which acts as a binder or carrier to hold the agent in the stent and/or modulate the release of the agent from the stent. The drug will be held within the reservoirs in the stent in a drug delivery matrix comprised of the drug and a polymeric or other material and optionally additives to regulate the drug release.

The therapeutic agent containing matrix can be disposed in the stent in various configurations, including within volumes defined by the stent, such as openings, holes, grooves, channels, or concave surfaces, as a reservoir of agent. When the therapeutic agent matrix is disposed within openings in the strut structure of the stent to form a reservoir, the openings may be partially or completely filled with matrix containing the therapeutic agent. The beneficial agent matrix when fixed to the stent is arranged such that it does not block access of fluid from the surrounding environment to the bioresorbable stent or otherwise appreciable change the bioresorbtion of the stent.

The beneficial agent matrix within the openings may be formed by one of a plurality of methods. One such method is described in US Patent Application Serial No. 10/668,125, filed on September 22, 2003. According to this method the matrix is loaded into the openings by forming a solution of polymer, drug, and solvent, and delivering the solution into the openings by a piezoelectric dispenser in a plurality of steps which form multiple individual or intermixing layers with different chemical and/or pharmacological properties.

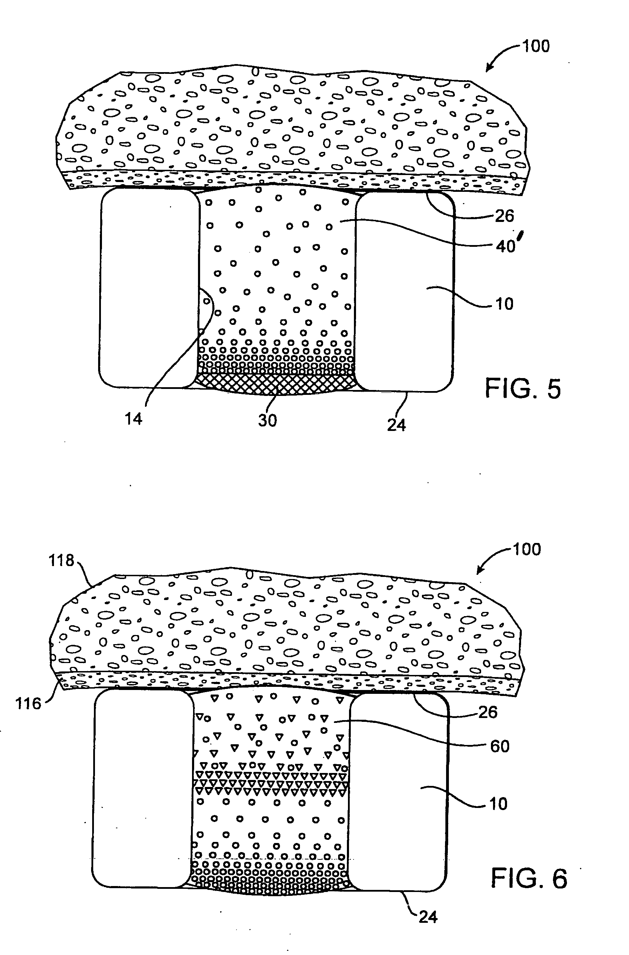

FIG. 5 is a cross section of one strut of the stent 10 and a blood vessel 100 illustrating one example of a through opening 14 arranged adjacent the vessel wall with a mural surface 26 abutting the vessel wall and a luminal surface 24 opposite the mural surface. The opening 14 of FIG. 3 contains a matrix 40' with a therapeutic agent illustrated by Os in the matrix. The luminal side 24 of the stent opening 14 is provided with a barrier layer 30. The barrier layer 30 erodes more slowly than the matrix 40' containing the therapeutic agent and thus, causes the therapeutic agent to be delivered primarily to the mural side 26 of the stent. The matrix 40' and therapeutic agent are arranged in a programmable manner to achieve a desire release rate and administration period. As can be seen in the example of FIG. 5, the concentration of the therapeutic agent (Os) is highest adjacent the barrier layer 30 of the stent 10 and lowest at the mural side 26 of the stent. This configuration in which the drug can be precisely arranged within the matrix allows the release rate and administration period to be selected and programmed to a particular application. The methods by which the drug can be precisely arranged within the matrix in the openings is a stepwise deposition process and is further described in U.S. Patent Application Serial No. 10/777,283, filed February 11, 2004 .

FIG. 6 is a cross section of a strut of the stent 10 having an opening 14 in which a polymer/drug matrix 60 includes a first drug illustrated by Os and second drug illustrated by ▼ s. The two drugs may be located in separate regions of the matrix or intermixed (as shown) to achieve different release profiles and administration periods for the two drugs.

Numerous other useful arrangements of the matrix and therapeutic agent can be formed to achieve different release rates including substantially linear release, substantially first order release, pulsitile release, or any other desired release. The arrangement of the polymer and agent in the matrix also controls the duration of release or administration period which may be a short release of 1-24 hours, moderate release of about 1 to about 7 days, or extended release of about 7 or more days, preferably about 30 days. Each of the areas of the matrix may include one or more agents in the same or different proportions from one area to the next. The matrix may be solid, porous, or filled with other drugs or excipients. The agents may be homogeneously disposed or heterogeneously disposed in different areas of the matrix.

When an anti-restenotic agent delivered by the method of the invention is paclitaxel, the total amount delivered (and loaded) is preferably between 2 micrograms and 50 micrograms. In one preferred embodiment, the amount of paclitaxel delivered will be between about 0.1 micrograms and about 15 micrograms on the first day, more preferably between about 0.3 micrograms and about 9 micrograms. Following day one, the paclitaxel will be delivered in a substantially linear fashion at a rate of about 0.025 micrograms to about 2.5 microgram per day for a minimum of 21 days, preferably about 0.2 to about 2 micrograms per day. It is envisioned that all the paclitaxel will be released from the stent in less than 60 days. The total amount of paclitaxel loaded onto the stent and released into the tissue in need of treatment is envisioned to be preferably in the range of about 1.5 micrograms to about 75 micrograms, preferably about 3 to about 30 micrograms. The above release rates for paclitaxel have been given for a standard stent of dimensions 3.0 mm in expanded diameter by 17 mm in length. Stents of other dimensions are envisioned to contain total drug loadings in similar respective proportions based on similar drug loading density or drug per unit length. In one example, the amount of paclitaxel released per day after day one is about 0.0003 to about 0.03 ug/mm² of tissue surface area, preferably about 0.0003 to about 0.01 ug/mm² of tissue surface area. In another example, the amount of paclitaxel released per day after day one is about 0.001 to about 0.2 ug/mm of stent length per day.

The methods disclosed preferably will result in sustained release of substantially all the drug loaded onto the stent in no longer than 180 days, preferably in no longer than 60 days, and most preferably in no longer than 35 days.

It is envisioned that all beneficial agent matrix will be bioresorbed in about 14 days to about one year, more preferably in about 30 days to about 90 days. It is also envisioned that stent structure will be bioresorbed in about 20 days to about 365 days, preferably about 30 days to about 180 days.

### Therapeutic Agents

The present invention relates to the use of anti-restenotic agents including paclitaxel, rapamycin, cladribine, and their derivatives, as well as other cytotoxic or cytostatic agents and microtubule stabilizing agents. The present disclosure may also be used to deliver other agents alone or in combination with anti-restenotic agents. Some of the other agents delivered either alone or in combination may be those that to reduce tissue damage after myocardial infarction, stabilize vulnerable plaque, promote angiogenesis, or reduce inflammatory response.

Other therapeutic agents disclosed may, for example, take the form of small molecules, peptides, lipoproteins, polypeptides, polynucleotides encoding polypeptides, lipids, protein-drugs, protein conjugate drugs, enzymes, oligonucleotides and their derivatives, ribozymes, other genetic material, cells, antisense oligonucleotides, monoclonal antibodies, platelets, prions, viruses, bacteria, eukaryotic cells such as endothelial cells, stem cells, ACE inhibitors, monocyte/macrophages and vascular smooth muscle cells. Such agents can be used alone or in various combinations with one another. For instance, anti-inflammatories may be used in combination with antiproliferatives to mitigate the reaction of tissue to the antiproliferative. The therapeutic agent may also be a pro-drug, which metabolizes into the desired drug when administered to a host. In addition, therapeutic agents may be pre-formulated as microcapsules, microspheres, microbubbles, liposomes, niosomes, emulsions, dispersions or the like before they are incorporated into the matrix. Therapeutic agents may also be radioactive isotopes or agents activated by some other form of energy such as light or ultrasonic energy, or by other circulating molecules that can be systemically administered.

Exemplary classes of therapeutic agents include antiproliferatives, antithrombins (i.e., thrombolytics), immunosuppressants, antilipid agents, anti-inflammatory agents, antineoplastics including antimetabolites, antiplatelets, angiogenic agents, anti-angiogenic agents, vitamins, antimitotics, metalloproteinase inhibitors, NO donors, nitric oxide release stimulators, anti-sclerosing agents, vasoactive agents, endothelial growth factors, beta blockers, AZ blockers, hormones, statins, insulin growth factors, antioxidants, membrane stabilizing agents, calcium antagonists (i.e., calcium channel antagonists), retinoids, anti-macrophage substances, antilymphocytes, cyclooxygenase inhibitors, immunomodulatory agents, angiotensin converting enzyme (ACE) inhibitors, anti-leukocytes, high-density lipoproteins (HDL) and derivatives, cell sensitizers to insulin, prostaglandins and derivatives, anti-TNF compounds, hypertension drugs, protein kinases, antisense oligonucleotides, cardio protectants, petidose inhibitors (increase blycolitic metabolism), endothelin receptor agonists, interleukin-6 antagonists, anti-restenotics, and other miscellaneous compounds.

Antiproliferatives include, without limitation, sirolimus, paclitaxel, actinomycin D, rapamycin, and cyclosporin.

Antithrombins include, without limitation, heparin, plasminogen, α ₂-antiplasmin, streptokinase, bivalirudin, and tissue plasminogen activator (t-PA).

Immunosuppressants include, without limitation, cyclosporine, rapamycin and tacrolimus (FK-506), sirolumus, everolimus, etoposide, and mitoxantrone.

Antilipid agents include, without limitation, HMG CoA reductase inhibitors, nicotinic acid, probucol, and fibric acid derivatives (e.g., clofibrate, gemfibrozil, gemfibrozil, fenofibrate, ciprofibrate, and bezafibrate).

Anti-inflammatory agents include, without limitation, salicylic acid derivatives (e.g., aspirin, insulin, sodium salicylate, choline magnesium trisalicylate, salsalate, dflunisal, salicylsalicylic acid, sulfasalazine, and olsalazine), para-amino phenol derivatives (e.g., acetaminophen), indole and indene acetic acids (e.g., indomethacin, sulindac, and etodolac), heteroaryl acetic acids (e.g., tolmetin, diclofenac, and ketorolac), arylpropionic acids (e.g., ibuprofen, naproxen, flurbiprofen, ketoprofen, fenoprofen, and oxaprozin), anthranilic acids (e.g., mefenamic acid and meclofenamic acid), enolic acids (e.g., piroxicam, tenoxicam, phenylbutazone and oxyphenthatrazone), alkanones (e.g., nabumetone), glucocorticoids (e.g., dexamethaxone, prednisolone, and triamcinolone), pirfenidone, and tranilast.

Antineoplastics include, without limitation, nitrogen mustards (e.g., mechlorethamine, cyclophosphamide, ifosfamide, melphalan, and chlorambucil), methylnitrosoureas (e.g., streptozocin), 2-chloroethylnitrosoureas (e.g., carmustine, lomustine, semustine, and chlorozotocin), alkanesulfonic acids (e.g., busulfan), ethylenimines and methylmelamines (e.g., triethylenemelamine, thiotepa and altretamine), triazines (e.g., dacarbazine), folic acid analogs (e.g., methotrexate), pyrimidine analogs (5-fluorouracil, 5-fluorodeoxyuridine, 5-fluorodeoxyuridine monophosphate, cytosine arabinoside, 5-azacytidine, and 2',2'-difluorodeoxycytidine), purine analogs (e.g., mercaptopurine, thioguanine, azathioprine, adenosine, pentostatin, cladribine, and erythrohydroxynonyladenine), antimitotic drugs (e.g., vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin), phenoxodiol, etoposide, and platinum coordination complexes (e.g., cisplatin and carboplatin).

Antiplatelets include, without limitation, insulin, dipyridamole, tirofiban, eptifibatide, abciximab, and ticlopidine.

Angiogenic agents include, without limitation, phospholipids, ceramides, cerebrosides, neutral lipids, triglycerides, diglycerides, monoglycerides lecithin, sphingosides, angiotensin fragments, nicotine, pyruvate thiolesters, glycerol-pyruvate esters, dibydoxyacetone-pyruvate esters and monobutyrin.

Anti-angiogenic agents include, without limitation, endostatin, angiostatin, fumagillin and ovalicin.

Vitamins include, without limitation, water-soluble vitamins (e.g., thiamin, nicotinic acid, pyridoxine, and ascorbic acid) and fat-soluble vitamins (e.g., retinal, retinoic acid, retinaldehyde, phytonadione, menaqinone, menadione, and alpha tocopherol).

Antimitotics include, without limitation, vinblastine, vincristine, vindesine, vinorelbine, paclitaxel, docetaxel, epipodophyllotoxins, dactinomycin, daunorubicin, doxorubicin, idarubicin, epirubicin, mitoxantrone, bleomycins, plicamycin and mitomycin.

Metalloproteinase inhibitors include, without limitation, TIMP-1, TIMP-2, TIMP-3, and SmaPI.

NO donors include, without limitation, L-arginine, amyl nitrite, glyceryl trinitrate, sodium nitroprusside, molsidomine, diazeniumdiolates, S-nitrosothiols, and mesoionic oxatriazole derivatives.

NO release stimulators include, without limitation, adenosine.

Anti-sclerosing agents include, without limitation, collagenases and halofuginone.

Vasoactive agents include, without limitation, nitric oxide, adenosine, nitroglycerine, sodium nitroprusside, hydralazine, phentolamine, methoxamine, metaraminol, ephedrine, trapadil, dipyridamole, vasoactive intestinal polypeptides (VIP), arginine, and vasopressin.

Endothelial growth factors include, without limitation, VEGF (Vascular Endothelial Growth Factor) including VEGF-121 and VEG-165, FGF (Fibroblast Growth Factor) including FGF-1 and FGF-2, HGF (Hepatocyte Growth Factor), and Ang1 (Angiopoietin 1).

Beta blockers include, without limitation, propranolol, nadolol, timolol, pindolol, labetalol, metoprolol, atenolol, esmolol, and acebutolol.

Hormones include, without limitation, progestin, insulin, the estrogens and estradiols (e.g., estradiol, estradiol valerate, estradiol cypionate, ethinyl estradiol, mestranol, quinestrol, estrond, estrone sulfate, and equilin).

Statins include, without limitation, mevastatin, lovastatin, simvastatin, pravastatin, atorvastatin, and fluvastatin.

Insulin growth factors include, without limitation, IGF-1 and IGF-2.

Antioxidants include, without limitation, vitamin A, carotenoids and vitamin E.

Membrane stabilizing agents include, without limitation, certain beta blockers such as propranolol, acebutolol, labetalol, oxprenolol, pindolol and alprenolol.

Calcium antagonists include, without limitation, amlodipine, bepridil, diltiazem, felodipine, isradipine, nicardipine, nifedipine, nimodipine and verapamil.

Retinoids include, without limitation, all-trans-retinol, all-trans-14-hydroxyretroretinol, all-trans-retinaldehyde, all-trans-retinoic acid, all-trans-3,4-didehydroretinoic acid, 9-cis-retinoic acid, 11-cis-retinal, 13-cis-retinal, and 13-cis-retinoic acid.

Anti-macrophage substances include, without limitation, NO donors.

Anti-leukocytes include, without limitation, 2-CdA, IL-1 inhibitors, anti-CD116/CD18 monoclonal antibodies, monoclonal antibodies to VCAM, monoclonal antibodies to ICAM, and zinc protoporphyrin.

Cyclooxygenase inhibitors include, without limitation, Cox-1 inhibitors and Cox-2 inhibitors (e.g., CELEBREX® and VIOXX®).

Immunomodulatory agents include, without limitation, immunosuppressants (see above) and immunostimulants (e.g., levamisole, isoprinosine, Interferon alpha, and Interleukin-2).

ACE inhibitors include, without limitation, benazepril, captopril, enalapril, fosinopril sodium, lisinopril, quinapril, ramipril, and spirapril.

Cell sensitizers to insulin include, without limitation, glitazones, P par agonists and metformin.

Antisense oligonucleotides include, without limitation, resten-NG.

Cardio protectants include, without limitation, VIP, pituitary adenylate cyclase-activating peptide (PACAP), apoA-I milano, amlodipine, nicorandil, cilostaxone, and thienopyridine.

Petidose inhibitors include, without limitation, omnipatrilat.

Anti-restenotics include, without limitation, include vincristine, vinblastine, actinomycin, epothilone, paclitaxel, and paclitaxel derivatives (e.g., docetaxel).

Miscellaneous compounds include, without limitation, Adiponectin.

While the invention has been described in detail with reference to the preferred embodiments thereof, it will be apparent to one skilled in the art that various changes and modifications can be made, without departing from the present invention as set out in the appended claims.

## Claims

1. An anti-restenotic drug for use in a method of reducing restenosis with a bioresorbable drug delivery stent, the method comprising:
providing a drug delivery bioresorbable stent (10, 40, 50) with a dosage of the anti-restenotic drug in a bioresorbable polymer matrix (40', 60) arranged within a plurality of openings (14) in the stent without coating an exterior surface of the stent with the anti-restenotic drug, wherein the bioresorbable material of the stent degrades more slowly than the bioresorbable polymer matrix and the bioresorbable polymer matrix is arranged in the plurality of openings such that it does not change the bioresorbtion of the stent; implanting the stent within an artery of a patient; and delivering the anti-restenotic drug from the bioresorbable polymer matrix in the plurality of openings in the stent, wherein the bioresorbable polymer matrix is arranged in the plurality of openings to release the anti-restenotic drug to the artery at a minimum release rate of 1 percent of the total dosage of the drug on the stent per day throughout an entire administration period from the time of implantation of the stent until the time that substantially all the drug is released from the stent.

2. The drug of Claim 1, wherein the anti-restenotic drug and bioresorbable polymer matrix are delivered to the openings by delivery of a solution containing the drug and polymer matrix in a plurality of steps-to create a matrix within the openings (14) which has a concentration gradient.

## Patentansprüche

1. Anti-restenotisches Arzneimittel zur Verwendung in einem Verfahren zum Vermindern von Restenose mit einem bioresorbierbaren Arzneimittel-Abgabe-Stent, wobei das Verfahren umfasst:
Bereitstellen eines bioresorbierbaren Arzneimittel-Abgabe-Stent (10, 40, 50) mit einer Dosis des anti-restenotischen Arzneimittels in einer bioresorbierbaren Polymermatrix (40', 60), die in einer Mehrzahl von Löchern (14) in dem Stent angeordnet ist, ohne eine äußere Oberfläche des Stents mit dem anti-restenotischen Arzneimittel zu beschichten, wobei das bioresorbierbare Material des Stents langsamer abgebaut wird als die bioresorbierbare Polymermatrix und die bioresorbierbare Polymermatrix so in der Mehrzahl von Löchern angeordnet ist, dass sie die Bioresorption des Stents nicht ändert;
Implantieren des Stents in einer Arterie eines Patienten; und
Abgeben des anti-restenotischen Arzneimittels aus der bioresorbierbaren Polymermatrix in der Mehrzahl von Löchern in dem Stent, wobei die bioresorbierbare Polymermatrix so in der Mehrzahl von Löchern angeordnet ist, dass das anti-restenotische Arzneimittel während der gesamten Verabreichungsdauer von der Implantation des Stents bis im Wesentlichen das gesamte Arzneimittel freigesetzt wurde mit einer Mindest-Freisetzungsrate von 1 Prozent der Gesamtdosis des Arzneimittels in dem Stent pro Tag an die Arterie abgegeben wird.

2. Arzneimittel nach Anspruch 1, wobei das anti-restenotische Arzneimittel und die bioresorbierbare Polymermatrix durch Einbringen in mehreren Schritten einer das Arzneimittel und die Polymermatrix enthaltenden Lösung in die Löcher eingebracht wird, wodurch in den Löchern eine Matrix erzeugt wird, die einen Konzentrationsgradienten aufweist.

## Revendications

1. Médicament anti-resténose pour son utilisation dans une méthode de réduction de la resténose avec un stent biorésorbable pour la libération d'un médicament; la méthode comprenant :
la fourniture d'un stent biorésorbable pour la libération d'un médicament (10, 40, 50) avec un dosage du médicament anti-resténose dans une matrice de polymère biorésorbable (40', 60) agencée au sein d'une pluralité d'ouvertures (14) dans le stent sans enrobage d'une surface extérieure du stent avec le médicament anti-resténose, le matériau biorésorbable du stent se dégradant plus lentement que la matrice de polymère biorésorbable et la matrice de polymère biorésorbable étant agencée dans la pluralité d'ouvertures de telle façon qu'elle ne change pas la biorésorption du stent ;
l'implantation du stent au sein d'une artère d'un patient ; et la libération du médicament anti-resténose à partir de la matrice de polymère biorésorbable dans la pluralité d'ouvertures dans le stent, la matrice de polymère biorésorbable étant agencée dans la pluralité d'ouvertures pour libérer le médicament anti-resténose dans l'artère à une vitesse de libération minimale de 1 pour cent du dosage total du médicament sur le stent par jour durant une période entière d'administration depuis le moment de l'implantation du stent jusqu'au moment où substantiellement la totalité du médicament est libérée du stent.

2. Médicament selon la revendication 1, dans lequel le médicament anti-resténose et la matrice de polymère biorésorbable sont libérés dans les ouvertures par libération d'une solution contenant le médicament et la matrice de polymère en une pluralité d'étapes pour créer une matrice au sein des ouvertures (14) qui présente un gradient de concentration.
